(19) **Europäisches Patentamt / European Patent Office / Office européen des brevets**

(11) **EP 4 398 866 B1**

(12) **EUROPEAN PATENT SPECIFICATION**

(45) Date of publication and mention
of the grant of the patent:
**20.08.2025 Bulletin 2025/34**

(21) Application number: **22772916.7**

(22) Date of filing: **01.09.2022**

(51) International Patent Classification (IPC):
*A61K 8/33* (2006.01)   *A61K 8/34* (2006.01)
*A61K 8/36* (2006.01)   *A61K 8/37* (2006.01)
*A61K 8/39* (2006.01)   *A61K 8/44* (2006.01)
*A61K 8/55* (2006.01)   *A61Q 1/10* (2006.01)

(52) Cooperative Patent Classification (CPC):
**A61K 8/375; A61K 8/33; A61K 8/342; A61K 8/361; A61K 8/39; A61K 8/442; A61K 8/55; A61Q 1/10**

(86) International application number:
**PCT/EP2022/074340**

(87) International publication number:
**WO 2023/036685 (16.03.2023 Gazette 2023/11)**

(54) **AQUEOUS COMPOSITION COMPRISING A FATTY DIALKYL ETHER, AN ANIONIC SURFACTANT WITH AT LEAST ONE CATIONIC COUNTERION AND A NONIONIC SURFACTANT**

WÄSSRIGE ZUSAMMENSETZUNG MIT EINEM FETTDIALKYLETHER, EINEM ANIONISCHEN TENSID MIT MINDESTENS EINEM KATIONISCHEN GEGENION UND EINEM NICHTIONISCHEN TENSID

COMPOSITION AQUEUSE COMPRENANT UN ÉTHER DE DIALKYLE GRAS, UN TENSIOACTIF ANIONIQUE AVEC AU MOINS UN CONTRE-ION CATIONIQUE ET UN TENSIOACTIF NON IONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2021 FR 2109490**

(43) Date of publication of application:
**17.07.2024 Bulletin 2024/29**

(73) Proprietor: **L'OREAL**
**75008 Paris (FR)**

(72) Inventors:
• **VAILLANT, Clémence**
**94152 CHEVILLY LARUE (FR)**

• **ILEKTI, Philippe**
**94152 CHEVILLY LARUE (FR)**

(74) Representative: **L'Oreal**
**Service D.I.P.I.**
**9, rue Pierre Dreyfus**
**92110 Clichy (FR)**

(56) References cited:
**EP-A1- 1 920 759**

• **DATABASE GNPD [online] MINTEL; 2 November 2012 (2012-11-02), ANONYMOUS: "Lengthening Mascara", XP055923147, retrieved from https://www.gnpd.com/sinatra/recordpage/1894303/ Database accession no. 1894303**

**Description**

**Technical field**

The present invention relates to the field of caring for and/or making up keratin materials, and is directed towards proposing compositions more particularly intended for making up the eyelashes or the eyebrows.

In general, compositions intended for making up keratin fibers, for example the eyelashes, aim to densify the thickness and the visual perception of the eyelashes and ultimately the gaze. These mascaras are described as aqueous or cream mascaras when they are formulated in an aqueous base, and anhydrous mascaras when they are formulated as a dispersion in an organic solvent medium.

A great diversity of cosmetic effects may be afforded by applying a mascara to keratin fibers and notably the eyelashes, for instance a volumizing, lengthening, thickening and more particularly charging makeup effect.

EP1920759 A1 discloses a cosmetic composition for coating eyelashes comprising a C10-C30 alkyl phosphate, a fatty alcohol ether of polyethylene glycol having an HLB <8 and water.

For obvious reasons, improving the textures of mascara which condition the manifestation of one or more makeup effects is a constant preoccupation of cosmetic formulators.

Indeed, a texture resulting in a very charging effect generally results in a relatively unesthetic appearance since the eyelashes are very often stuck together. A texture which leaves the consumer in control of their level of charge and also the separation of the eyelashes is therefore sought.

An excessively fluid texture deposits little on the eyelashes, whereas an excessively thick texture is not deposited on the eyelashes. On the other hand, a mascara texture that is creamy (intermediate between fluid and too thick) will offer the consumer a pleasantness on application and a charging makeup result on the eyelashes. Thus, the texture has a strong impact on the capacity of the mascara to be deposited on the eyelashes and to provide the desired makeup result. For these reasons, it becomes essential to finely control the texture. However, mascara textures have the drawback of changing too rapidly. Thus, the consumer sometimes observes significant differences in texture and therefore in makeup result for one and the same mascara sample during its use.

Thus, a first objective of the present invention is to obtain mascara compositions which have a creamy texture making it possible to obtain a satisfactory and stable charging effect.

A second objective of the present invention is to obtain mascara compositions which can be easily applied to keratin fibers, with an aesthetic appearance and which are comfortable for the consumer, notably smooth, supple compositions which are easy to apply.

A third objective of the present invention is to obtain mascara compositions which have good makeup properties on keratin fibers, notably the eyelashes, with a good volumizing effect, a good extension effect, a good thickening effect, a deposit which has good mechanical properties and a good charging effect, while at the same time obtaining good separation of said keratin fibers.

In the course of its research, the applicant has discovered, unexpectedly, that the objectives as defined above are achieved by using a composition comprising, notably in a physiologically acceptable medium:

(1) at least one fatty dialkyl ether of formula (I) below:

$$[Chem 1] \quad R_1\text{-O-}R_2 \qquad (I)$$

wherein $R_1$ and $R_2$, which may be identical or different, denote a linear and saturated alkyl radical having a number of carbon atoms ranging from 16 to 20; and
(2) at least one anionic surfactant comprising (3) at least one cationic counterion, preferably at least one anionic surfactant in a form partially or totally neutralized by said cationic counterion; and
(4) at least one nonionic surfactant with an HLB of less than or equal to 8;
(5) water.

Unexpectedly, the inventors have in fact noted that the formulation comprising (1) at least one fatty dialkyl ether of formula (I) as defined below, (2) at least one anionic surfactant comprising at least one cationic counterion (3), preferably in a form partially or totally neutralized by said cationic counterion (3), (4) at least one nonionic surfactant with an HLB of less than or equal to 8, and (5) water makes it possible to obtain compositions which have a creamy texture making it possible to obtain a satisfactory and stable charging effect. This formulation also makes it possible to obtain mascara compositions which can be easily applied to keratin fibers, with an aesthetic appearance and which are comfortable for the consumer, notably smooth, supple compositions which are easy to apply. This formulation also makes it possible to obtain mascara compositions which have good makeup properties on keratin fibers, notably the eyelashes, with a good volumizing effect, a good extension effect, a good thickening effect, a deposit which has good mechanical properties and a good charging effect, while at the same time obtaining good separation of said keratin fibers.

This discovery forms the basis of the invention.

## Subjects of the invention

Thus, according to one of its aspects, the present invention relates to a composition comprising, notably in a physiologically acceptable medium:

(1) at least one fatty dialkyl ether of formula (I) below:

[Chem 1] $R_1$-O-$R_2$ (I)

wherein $R_1$ and $R_2$, which may be identical or different, denote a linear and saturated alkyl radical having a number of carbon atoms ranging from 16 to 20; and

(2) at least one anionic surfactant comprising (3) at least one cationic counterion, preferably said anionic surfactant in a form partially or totally neutralized by said cationic counterion; and

(4) at least one nonionic surfactant with an HLB of less than or equal to 8;

(5) water.

A second subject of the present invention is a cosmetic process for making up and/or caring for human keratin materials, such as the skin, notably the area around the eyes, the contour of the eyelashes, the contour of the eyebrows; keratin fibers such as the eyelashes and the eyebrows, consisting in applying to said keratin materials at least one composition as defined above.

## Definitions

In the context of the present invention, the term "keratin material" is notably intended to mean the skin, notably the contour of the eyes, the contour of the eyelashes, the contour of the eyebrows; keratin fibers such as the eyelashes and the eyebrows. For the purposes of the present invention, this term "keratin fibers" also extends to synthetic false eyelashes.

The term "physiologically acceptable" means compatible with the skin and/or its integuments, which has a pleasant color, odor and feel, and which does not cause any unacceptable discomfort (stinging or tautness) liable to discourage the consumer from using this composition.

## Fatty dialkyl ether

A composition according to the invention comprises at least one fatty dialkyl ether of formula (I) below:

[Chem 1] $R_1$-O-$R_2$ (I)

wherein $R_1$ and $R_2$, which may be identical or different, denote a linear and saturated alkyl radical having a number of carbon atoms ranging from 16 to 20.

A composition according to the invention may comprise at least 3.0% by weight of fatty dialkyl ether(s) of formula (I), preferably at least 5.0% by weight, relative to the total weight of the composition.

According to one particularly preferred embodiment of the invention, the fatty dialkyl ether(s) of formula (I) are present in the composition preferably in a content ranging from 5.0% to 35.0% by weight, or even more preferentially from 8.0% to 25.0% by weight, relative to the total weight of the composition.

This or these fatty dialkyl ether(s) is (are) used during the preparation of a composition according to the invention, in an individualized form or in the form of a mixture exclusively comprising fatty dialkyl ethers of formula (I).

In one preferred embodiment, the one or more fatty dialkyl ethers are solid at ambient temperature (25° C) and atmospheric pressure (760 mmHg). They preferably have a melting point above 50° C.

The melting point may be measured by any known method and in particular using a differential scanning calorimeter (DSC).

In particular, the fatty dialkyl ether of formula (I) according to the invention is chosen from

- the dicetyl ether having the structure:

$$CH_3(CH_2)_{15}O(CH_2)_{15}CH_3$$

- the distearyl ether having the structure:

$$CH_3(CH_2)_{17}O(CH_2)_{17}CH_3;$$

and mixtures thereof.

According to one particularly preferred embodiment of the invention, the fatty dialkyl ether of formula (I) is distearyl ether, such as the products sold under the trade name Cosmacol SE® by Sasol or under the trade name Cutina STE® by BASF.

**Anionic surfactant**

As specified above, the composition according to the invention contains at least one anionic surfactant comprising at least one cationic counterion.

According to one particular form of the invention, the surfactant can be partially neutralized by said cationic counterion.

According to another particular form of the invention, the surfactant can be in the form of a mixture, one portion of which is neutralized and the other non-neutralized.

According to one particularly preferred form of the invention, the surfactant is totally neutralized by said cationic counterion and thus forms a salt.

For the purposes of the present invention, the term "surfactant" is understood to mean **an amphiphilic chemical compound, that is to say a compound exhibiting, in its structure, two different polarities. Generally, one is lipophilic** (soluble or dispersible in an oily phase). The other is hydrophilic (soluble or dispersible in water). Surfactants are characterized by the value of their HLB (hydrophilic-lipophilic balance), the HLB being the ratio of the hydrophilic part to the lipophilic part in the molecule. The term "HLB" is well known to those skilled in the art and is described, for example, in "The HLB System. A time-saving guide to Emulsifier Selection" (published by ICI Americas Inc; 1984).

According to one preferential form of the invention, the anionic surfactants in accordance with the invention have an HLB greater than or equal to 8. The HLB of the anionic surfactant(s) used according to the invention may be determined by the Griffin method.

The term "anionic surfactant" is understood to mean **any** negatively charged **amphiphilic molecule.**

The anionic surfactant(s) in accordance with the invention is (are) preferably chosen from:

i. $C_{12}$-$C_{20}$ monoalkyl phosphates;

ii. alkyl sulfates, and in particular alkyl ether sulfates, alkylamido ether sulfates, alkylaryl polyether sulfates, monoglyceride sulfates;

iii. alkylsulfonates, alkylamidesulfonates, alkylarylsulfonates, -olefinsulfonates, paraffinsulfonates;

iv. alkyl sulfosuccinates, alkyl ether sulfosuccinates, alkylamide sulfosuccinates; alkyl sulfosuccinamates;

v. acyl sarcosinates, acyl glutamates, acyl isethionates, N-acyl taurates and acyl lactylates;

vi. fatty acids, in particular palmitic acid or stearic acid, coconut oil acid or hydrogenated coconut oil acid;

vii. mixtures thereof.

The anionic surfactant(s) in accordance with the invention is (are) preferably present in a total content of greater than or equal to 2% by weight relative to the total weight of the composition, preferably ranging from 3% to 15% and better still from 3% to 12% by weight, relative to the total weight of the composition.

The cationic counterion(s) is (are) chosen from a cation of mineral origin, in particular chosen from alkali metal (sodium, potassium, lithium) cations, alkaline-earth metal (magnesium, calcium) cations, the ammonium ion ($NH_4^+$) or organic cations.

Said organic cationic counterion(s) is (are) preferably chosen from amines and aminoalcohols.

According to one particular form of the invention, said organic cationic counterion(s) comprise(s) a primary (poly)hydroxyalkylamine.

The term "primary (poly)hydroxyalkylamine" in particular means a primary dihydroxyalkylamine, it being understood that the term "primary" means a primary amine function, i.e. $-NH_2$, and the alkyl group being a linear or branched $C_1$-$C_8$ and preferably a branched $C_4$ hydrocarbon-based chain, such as 1,3-dihydroxy-2-methylpropyl (also called aminomethylpropanediol or AMPD).

The total content of cationic counterion(s) is preferably greater than or equal to 0.1%, more preferentially inclusively between 0.1% and 4.0% by weight, better still between 0.2% and 3.0% by weight, with respect to the total weight of the composition.

According to one particularly preferred form, the anionic surfactant(s) in accordance with the invention is (are) preferably chosen from:

a) $C_{12}$-$C_{20}$ monoalkyl phosphates;

b) $C_{12}$-$C_{20}$ acylglutamates;

c) fatty acids containing from 14 to less than 20 carbon atoms;

d) and mixtures thereof.

a) $C_{12}$-$C_{20}$ Monoalkyl phosphates

The designation "monoalkyl" is intended to mean that the phosphate element is linked to a single $C_{12}$-$C_{20}$ alkyl chain, unless otherwise specified. The monoalkyl phosphate(s) (including phosphine oxide(s)) that may be used in the compositions according to the present application is/are chosen from $C_{14}$-$C_{20}$ and preferably $C_{16}$-$C_{18}$ monoalkyl phosphates and mixtures thereof..

Preferably, they are chosen from monocetyl phosphate, monostearyl phosphate and monocetearyl phosphate.

In particular, use will be made of the anionic surfactant in its form neutralized by the cationic counterion potassium, namely the potassium salt of monocetyl phosphate having the INCI name Potassium Cetyl Phosphate, for example sold under the names Amphisol K® (DSM Nutritional Products), Amphisol A® (DSM Nutritional Products), Arlatone MAP® (Uniqema), Crodafos MCA® (Croda).

b) $C_{12}$-$C_{20}$ Acylglutamates

The one or more acylglutamates have at least one $C_{12}$-$C_{20}$ acyl chain. They are preferably chosen from lauroyl glutamate, myristoyl glutamate, palmitoyl glutamate, stearoyl glutamate, and a mixture or mixtures thereof.

According to one particular embodiment, use will be made of the anionic surfactant in its form neutralized by the cationic counterion sodium, namely the sodium salt of stearoyl glutamate having the INCI name Sodium Stearoyl Glutamate, more particularly that sold by Ajinomoto under the reference Amisoft HS 11®.

c) Fatty acid having from 14 to less than 20 carbon atoms

The fatty acid according to the invention preferably comprises from 14 to less than 20 carbon atoms. According to one particularly preferred embodiment, the number of carbon atoms ranges from 14 to 18.

In particular, the fatty acid(s) according to the invention are chosen from linear fatty acids, saturated fatty acids and mixtures thereof.

According to one particularly advantageous embodiment of the invention, the fatty acid of the anionic surfactant is linear and saturated.

According to a particular embodiment of the invention, the fatty acid(s) are chosen from palmitic acid, stearic acid and mixtures thereof, and preferably comprise at least stearic acid having the INCI name Stearic Acid.

According to another embodiment of the invention, the composition contains at least the anionic surfactant stearic acid in its form neutralized by the cationic counterion aminomethylpropanediol or AMPD.

**Nonionic surfactants with an HLB ≤ 8**

The composition according to the invention comprises, as co-surfactants, one or more nonionic surfactants with an HLB of less than or equal to 8, measured at 25° C.

The term "HLB" (Hydrophilic Lipophilic Balance) is well known to those skilled in the art, and denotes the hydrophilic-lipophilic balance of a surfactant determined at 25° C in the Griffin sense. The term "hydrophilic-lipophilic balance (HLB)" means the equilibrium between the size and strength of the hydrophilic group and the size and strength of the lipophilic group of the surfactant. The HLB value according to Griffin is defined in J. Soc. Cosm. Chem. 1954 (volume 5), pages 249-256.

The term "nonionic surfactant" is intended to mean **any neutral amphiphilic molecule** not comprising an ionic charge.

More preferentially, use will be made of a nonionic surfactant with an HLB ≤ 8, measured at 25° C, chosen from

- fatty alcohols, notably $C_{14}$-$C_{30}$, and more preferentially $C_{14}$-$C_{24}$, more particularly $C_{14}$-$C_{20}$, fatty alcohols;

- ethers of a fatty alcohol, notably a $C_8$-$C_{24}$ fatty alcohol, and of polyethylene glycol (PEG) comprising from 1 to 10 ethylene glycol units, preferably from 2 to 10 ethylene glycol units;

- esters of fatty acids, notably $C_8$-$C_{24}$ and preferably $C_{16}$-$C_{22}$ fatty acids, and of glycerol;

and mixtures thereof.

### i) Fatty alcohol

A composition can therefore comprise a single fatty alcohol according to the invention or several distinct fatty alcohols with an HLB ≤ 8 measured at 25° C.

If several distinct fatty alcohols are present, they can be added separately during the preparation of the composition and the mixture thereof can then be formed in situ. They can also be used in the form of a mixture which is already commercially available and in which the weight proportion and the degree of purity of each of the fatty alcohols are controlled. In other words, the composition of these mixtures is faithfully reproducible, as opposed to mixtures of fatty alcohols generated via synthesis from starting materials derived from complex mixtures.

According to a preferred embodiment, the fatty alcohol is solid at ambient temperature.

The fatty alcohol(s) is (are) in particular chosen from linear or branched, saturated or unsaturated $C_{14}$-$C_{30}$, preferably $C_{14}$-$C_{24}$, and even better still $C_{14}$-$C_{20}$, fatty alcohols.

Preferably, the fatty alcohol according to the invention is chosen from cetyl alcohol ($C_{16}$), stearyl alcohol ($C_{18}$) and mixtures thereof (also known as "cetearyl alcohol").

According to one particular embodiment, the fatty alcohol is in the form of a mixture of cetyl alcohol and stearyl alcohol. Such a mixture is notably sold under the name Lannette O OR/MB® by BASF.

According to another particular embodiment, the fatty alcohol is cetyl alcohol, such as the product sold under the name Lanette 16® by BASF.

According to one preferential embodiment of the invention, the fatty alcohol nonionic surfactant(s) is (are) present in amounts ranging from 1.0% to 10.0% by weight, relative to the total weight of the composition, preferably from 2.0% to 10% by weight, and even more particularly from 3% to 8% by weight, relative to the total weight of the composition.

### ii) Ethers of a fatty alcohol and of polyethylene glycol (or PEG)

A composition of the invention can comprise one or more ether(s) of a $C_8$-$C_{24}$ fatty alcohol and of polyethylene glycol comprising from 1 to 10 ethylene glycol units with an HLB ≤ 8 measured at 25° C.

The ether or the ether(s) of a $C_8$-$C_{24}$ fatty alcohol and of polyethylene glycol comprising from 1 to 10 ethylene glycol units with an HLB ≤ 8, measured at 25° C, is (are) preferably chosen from ethers of $C_{12}$-$C_{22}$, more preferentially $C_{16}$-$C_{18}$, fatty alcohols and of polyethylene glycol comprising from 1 to 10 ethylene glycol units, preferably from 2 to 6 ethylene glycol units.

Particularly preferably, use is made of the compound bearing the INCI name Steareth-2 (polyethylene glycol(2) stearyl ether) with an HLB equal to 5; this product is for example sold under the names BRIJ 72® (Uniqema) and Volpo S2® (Croda).

The nonionic surfactant(s) which is an (are) ether(s) of a fatty alcohol and of polyethylene glycol with an HLB value at 25° C of less than or equal to 8 is (are) preferably present in a content of from 1.0% to 10% by weight, relative to the total weight of the composition, preferably from 2.0% to 10% by weight, and even more particularly from 3% to 8% by weight, relative to the total weight of the composition.

### iii) Esters of fatty acids and of glycerol

A composition of the invention can comprise one or more esters of fatty acids, notably $C_8$-$C_{24}$ and preferably $C_{16}$-$C_{22}$ fatty acids, and of glycerol with an HLB value at 25° C of less than or equal to 8.

Preferably, the esters of fatty acids, notably $C_8$-$C_{24}$ and preferably $C_{16}$-$C_{22}$ fatty acids, and of glycerol with an HLB value at 25° C of less than or equal to 8 are chosen from those of formula (II) below:.

[Chem 2] (ALK-[C(X)]x-O)y-Gly       (II)

wherein ALK is a $C_7$-$C_{23}$, preferably $C_{11}$-$C_{21}$ and more preferentially $C_{13}$-$C_{17}$ alkyl group;

X being O or S, preferably O;

x is equal to 0 or 1 and preferably x is equal to 1;

y is greater than or equal to 1, particularly between 1 and 3, preferably equal to 1;

Gly is a single glycerol group, preferentially *$CH_2OH$-CHOH-$CH_2OH$, wherein at least one of the -OH functions, and preferentially a single -OH function, is substituted with an ALK-[C(X)]x-O group, and preferably ALK-[C(O)]x-O group.

The alkyl chain of the $C_{12}$-$C_{20}$ fatty acid may be linear or branched, and saturated or unsaturated, preferably linear and saturated.

The term "fatty acid" should be understood as meaning a fatty monoacid.

The nonionic surfactant which is an ester of a fatty acid and of glycerol with an HLB value at 25° C of less than 8 is preferably glyceryl monostearate.

The nonionic surfactant(s) which is a (are) nonionic ester(s) of a fatty acid and of glycerol with an HLB value at 25° C of less than or equal to 8 are preferably present in a content of from 1.0% to 10% by weight, relative to the total weight of the composition, preferably from 2.0% to 10% by weight, and even more particularly from 3% to 8% by weight, relative to the total weight of the composition.

### Aqueous phase

The composition according to the invention comprises water, and notably an aqueous phase.

The term "aqueous phase" is intended to mean a phase comprising water and also optionally all the solvents and ingredients that are water-soluble or water-miscible (water-miscibility greater than 50% by weight at 25° C), for instance lower monoalcohols containing from 1 to 5 carbon atoms such as ethanol or isopropanol, glycols containing from 3 to 8 carbon atoms such as propylene glycol, 1,3-butylene glycol, caprylyl glycol, pentylene glycol and dipropylene glycol.

The aqueous phase may contain a demineralized water or alternatively a floral water such as cornflower water and/or a mineral water such as Vittel water, Lucas water or La Roche Posay water and/or a spring water.

In particular, a composition according to the invention comprises at least 30.0% by weight, better still at least 40.0% by weight, more particularly at least 45% by weight, and even more particularly a content of from 45% to 65% by weight of water, relative to the total weight of the composition.

### Other components

In addition to the abovementioned compounds, a composition according to the invention may of course comprise secondary ingredients.

### a) Waxes

Thus, a composition according to the invention may also comprise at least one wax.

As specified in the preamble, for the purposes of the invention, the term "waxes" refers to lipophilic compounds, which are solid at ambient temperature (25° C) and at atmospheric pressure (760 mmHg), with a reversible solid/liquid change of state, which have a melting point of greater than or equal to 40° C, which may be up to 120° C.

It is recalled that, for the purposes of the invention, the waxes to which the abovementioned amount limitation relates are distinct from those capable of being embodied by the component which is a linear fatty monoether (I) according to the invention and additional fatty alcohol components as defined below.

This limitation relates more particularly to waxes made up of complex mixtures which are notably described in Ullmann's Encyclopedia of Industrial Chemistry 2015, Wiley-VCH Verlag GmbH & Co. KGaA.

Such waxes may notably be natural, but may also be synthetic.

The term "natural" wax is intended to denote any wax which pre-exists in nature or which can be converted, extracted or purified from natural compounds which exist in nature.

**EP 4 398 866 B1**

Among natural waxes, mention may notably be made of waxes termed fossil waxes, including those of petroleum origin, such as ozokerite, pyropissite, microcrystalline waxes also known as paraffins - including crude or gatsch waxes, gatsch raffinates, de-oiled gatsch, soft waxes, semirefined waxes, filtered waxes, refined waxes - and microcrystalline waxes termed microwaxes, including bright stock gatsch. The fossil waxes also contain lignite, also known as montan wax, or peat wax.

As natural waxes other than fossil waxes, mention may be made of plant waxes.

As examples of plant waxes, mention may be made of carnauba wax, candelilla wax, ouricury wax, sugarcane wax, jojoba wax, Trithrinax campestris wax, raffia wax, alfalfa wax, wax extracted from Douglas fir, sisal wax, flax wax, cotton wax, Batavia dammar wax, cereal wax, tea wax, coffee wax, rice wax, palm wax, Japan wax, mixtures thereof and derivatives thereof.

As a natural wax other than plant waxes, mention may be made of beeswax.

The term "synthetic" wax is intended to denote waxes of which the synthesis requires one or more chemical reactions performed by a human being.

Among the synthetic waxes, semi-synthetic waxes and totally synthetic waxes can be distinguished. Synthetic waxes may be waxes obtained via a Fischer-Tropsch process, constituted for example of paraffins with a number of carbon atoms ranging from 20 to 50 or waxes of polyolefins, for example homopolymers or copolymers of ethylene, of propene or butene, or even longer-chain -olefins. The latter can be obtained by thermomechanical degradation of polyethylene plastic, by the Ziegler process, by high-pressure processes, or else via processes catalyzed by metallocene species. These waxes may be crystallizable, partially crystallizable or amorphous. The abovementioned synthetic waxes are generally apolar and can be chemically treated to obtain polar waxes, for example via one or more of the following reactions: air oxidation, grafting, esterification, neutralization with metal soaps, amidation, direct copolymerizations or addition reactions.

In this case also, their composition may be constituted of a mixture of ingredients since the fatty-chain lengths are not well defined, thus forming a mixture of compounds having different fatty-chain lengths and for which it is difficult for manufacturers to guarantee perfect reproducibility from one production batch to another.

For the purposes of the invention, a multicomponent wax denotes a wax constituted of a mixture of several ingredients, either such that it exists naturally like natural waxes, or such that it is formed during the process of industrial synthesis of these materials.

According to one particularly preferred form, the composition of the invention comprises at least one wax chosen from carnauba wax, beeswax and mixtures thereof.

In particular, a composition according to the invention comprises the wax(es) in a content of less than 30.0%, preferably less than 20.0%, notably ranging from 2% to 18% by weight of wax, more preferentially from 3% to 15% by weight, relative to the total weight of the composition.

## b) Liquid fatty phase

A composition according to the invention may also comprise a liquid fatty phase.

Such a liquid fatty phase is an organic phase that is liquid at ambient temperature (25° C) and at atmospheric pressure (760 mmHg), non-aqueous and water-immiscible.

The composition(s) according to the invention may comprise at least one nonvolatile oil, chosen notably from nonvolatile hydrocarbon-based oils.

The term "hydrocarbon-based oil" refers to an oil mainly containing carbon and hydrogen atoms and possibly one or more functions chosen from hydroxyl, ester, ether and carboxylic functions.

The term "nonvolatile oil" refers to an oil that remains on the skin or the keratin fiber at ambient temperature and atmospheric pressure for at least several hours, and that notably has a vapor pressure of less than 2.66 Pa, preferably less than 0.13 Pa. By way of example, the vapor pressure may be measured according to the static method or via the effusion method by isothermal thermogravimetry, depending on the vapor pressure (standard OECD 104).

Nonvolatile hydrocarbon-based oils that may notably be mentioned include:

- linear or branched hydrocarbons, of mineral or synthetic origin, such as liquid paraffins and derivatives thereof, petroleum jelly, polydecenes, polybutenes or polyisobutenes, which are optionally hydrogenated such as Parleam, or squalane;

- triglycerides constituted of fatty acid esters of glycerol, in particular the fatty acids of which may have chain lengths ranging from $C_4$ to $C_{36}$, and notably from $C_{18}$ to $C_{36}$, these oils possibly being linear or branched, and saturated or unsaturated; these oils may notably be heptanoic or octanoic triglycerides, wheatgerm oil, sunflower oil, grapeseed oil, sesame oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil,

8

marrow oil, blackcurrant oil, evening primrose oil, millet oil, barley oil, quinoa oil, rye oil, safflower oil, candlenut oil, passionflower oil or musk rose oil; shea oil; or alternatively caprylic/capric acid triglycerides, for instance those sold by Stéarineries Dubois or those sold under the names Miglyol 810®, 812® and 818® by Dynamit Nobel;

- aliphatic hydrocarbon-based esters of formula RCOOR' wherein RCOO represents a carboxylic acid residue including from 2 to 40 carbon atoms, and R' represents a hydrocarbon-based chain containing from 2 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, heptanoates, and notably isostearyl heptanoate, octanoates, cetyl octanoate, tridecyl octanoate, hexyl laurate, neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, isotridecyl isononanoate and octyl isononanoate, oleyl erucate, isopropyl lauroyl sarcosinate;

- polyesters obtained by condensation of unsaturated fatty acid dimer and/or trimer and of diol, such as those described in patent application FR 0 853 634, in particular such as of dilinoleic acid and of 1,4-butanediol. Mention may notably be made in this respect of the polymer sold by Biosynthis under the name Viscoplast 14436H® (INCI name: Dilinoleic acid/butanediol copolymer) or copolymers of polyols and of diacid dimers, and esters thereof, such as Hailuscent ISDA®,

- esters of fatty acids having a total carbon number ranging from 35 to 70, such as pentaerythrityl tetrapelargonate;

- aromatic esters such as tridecyl trimellitate, $C_{12}$-$C_{15}$ alcohol benzoate, the 2-phenylethyl ester of benzoic acid, and butyloctyl salicylate,

- esters and polyesters of diol dimer and of monocarboxylic or dicarboxylic acid, such as esters of diol dimer and of fatty acid and esters of diol dimer and of dicarboxylic acid dimer, such as Lusplan DD-DA5® and Lusplan DD-DA7® sold by Nippon Fine Chemical and described in patent application US 2004175338;

- and mixtures thereof.

A composition according to the invention may comprise a content of nonvolatile oil of less than 8% by weight, preferably less than 5% by weight and preferentially less than 2% by weight of nonvolatile oil, relative to the total weight of the composition. According to a preferred embodiment, the composition is free of nonvolatile oil.

Volatile oils

A composition according to the invention may comprise at least one volatile oil.
According to one particular embodiment of the invention, the composition comprises less than 5.0% by weight of volatile oil(s), or even less than 2.0%, relative to the total weight of the composition.
In one particularly preferred embodiment of the invention, the composition is free of volatile oil.
The term "composition free of volatile oil" refers to any composition comprising less than 1% by weight of volatile oil, or even less than 0.5% by weight relative to the total weight of the composition, or even which is totally free of volatile oil.
For the purposes of the invention, the term "volatile oil" refers to any oil that is capable of evaporating on contact with the skin in less than one hour, at ambient temperature and atmospheric pressure. The volatile oil is a volatile cosmetic compound, which is liquid at ambient temperature, notably having a non-zero vapor pressure, at ambient temperature and atmospheric pressure, notably having a vapor pressure ranging from 2.66 Pa to 40 000 Pa, in particular ranging from 2.66 Pa to 13 000 Pa and more particularly ranging from 2.66 Pa to 1300 Pa.
These oils may be hydrocarbon-based oils.
The volatile hydrocarbon-based oils may be chosen from hydrocarbon-based oils containing from 8 to 16 carbon atoms, and notably branched $C_8$-$C_{16}$ alkanes, for instance $C_8$-$C_{16}$ isoalkanes of petroleum origin (also known as isoparaffins), for instance isododecane (also known as 2,2,4,4,6-pentamethylheptane), isodecane and isohexadecane, and for example the oils sold under the trade names Isopar or Permethyl, branched $C_8$-$C_{16}$ esters, isohexyl neopentanoate, and mixtures thereof. Other volatile hydrocarbon-based oils, for instance petroleum distillates, notably those sold under the name Shell Solt by Shell, may also be used. The volatile solvent is preferably chosen from volatile hydrocarbon-based oils containing from 8 to 16 carbon atoms, and mixtures thereof.

### d) Colorant

According to a specific embodiment of the invention, the composition of the invention comprises at least one colorant, which is synthetic, natural or of natural origin.

The colorant may be chosen from coated or uncoated pigments, water-soluble dyes, liposoluble dyes, and mixtures thereof.

Pigments

The term "pigments" is understood to mean white or colored and inorganic or organic particles which are insoluble in an aqueous medium and which are intended to color and/or opacify the resulting composition and/or deposit.

According to a specific embodiment, the pigments used according to the invention are chosen from inorganic pigments.

The term "inorganic pigment" is understood to mean any pigment which satisfies the definition of Ullmann's Encyclopedia in the chapter "Pigments, Inorganic". Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminum powder and copper powder. The following mineral pigments may also be used: $Ta_2O_5$, $Ti_3O_5$, $Ti_2O_3$, $TiO$, $ZrO_2$ as a mixture with $TiO_2$, $ZrO_2$, $Nb_2O_5$, $CeO_2$, $ZnS$.

The size of the pigment that is useful in the context of the present invention is generally greater than 100 nm and may range up to 10 $\mu$m, preferably from 200 nm to 5 $\mu$m and more preferentially from 300 nm to 1 $\mu$m.

According to a particular form of the invention, the pigments have a size characterized by a D[50] greater than 100 nm and possibly ranging up to 10 $\mu$m, preferably from 200 nm to 5 $\mu$m and more preferentially from 300 nm to 1 $\mu$m.

The sizes are measured by static light scattering using a commercial MasterSizer 3000® particle size analyzer from Malvern, which makes it possible to determine the particle size distribution of all of the particles over a wide range which may extend from 0.01 $\mu$m to 1000 $\mu$m. The data are processed on the basis of the conventional Mie scattering theory. This theory is the most suitable for size distributions ranging from the submicronic to multimicronic; it makes it possible to determine an "effective" particle diameter. This theory is notably described in the publication by Van de Hulst, H.C., Light Scattering by Small Particles, Chapters 9 and 10, Wiley, New York, 1957.

D[50] represents the maximum size that 50% by volume of the particles have.

In the context of the present invention, the mineral pigments are more particularly iron oxide and/or titanium dioxide. By way of example, mention may more particularly be made of titanium dioxides and iron oxides.

As mineral pigments that may be used in the invention, mention may also be made of nacres.

The term "nacres" should be understood as meaning colored particles of any form, which may or may not be iridescent, notably produced by certain molluscs in their shell, or alternatively synthesized, and which have a color effect via optical interference.

The nacres can be chosen from pearlescent pigments, such as titanium oxide-coated mica covered with an iron oxide, titanium oxide-coated mica covered with bismuth oxychloride, titanium oxide-coated mica covered with chromium oxide, titanium oxide-coated mica covered with an organic dye and also pearlescent pigments based on bismuth oxychloride. They can also be mica particles, at the surface of which are superimposed at least two successive layers of metal oxides and/or of organic colorants.

According to a specific embodiment, the pigments used according to the invention are chosen from inorganic pigments.

Examples of nacres that may also be mentioned include natural mica covered with titanium oxide, with iron oxide, with natural pigment or with bismuth oxychloride.

The nacres can more particularly have a yellow, pink, red, bronze, orangey, brown, gold and/or coppery color or glint.

Mention may also be made, among the pigments that may be used according to the invention, of those having an optical effect different from a simple conventional coloring effect, that is to say a unified and stabilized effect such as is produced by conventional colorants, such as, for example, monochromatic pigments. For the purposes of the invention, the term "stabilized" means lacking the effect of variability of the color with the angle of observation or in response to a temperature change.

For example, this material may be chosen from particles with a metallic tint, goniochromatic coloring agents, diffractive pigments, thermochromic agents, optical brighteners, and also fibers, notably interference fibers. Needless to say, these various materials may be combined in order simultaneously to afford two effects, or even a novel effect in accordance with the invention.

According to a particular embodiment, the composition according to the invention comprises at least one uncoated pigment.

According to another particular embodiment, the composition according to the invention comprises at least one pigment coated with at least one lipophilic or hydrophobic compound.

This type of pigment is particularly advantageous. Insofar as they are treated with a hydrophobic compound, they show predominant affinity for an oily phase, which can then convey them.

The coating can also comprise at least one additional nonlipophilic compound.

For the purposes of the invention, the "coating" of a pigment according to the invention generally denotes the total or partial surface treatment of the pigment with a surface agent, absorbed on, adsorbed on or grafted to said pigment.

The surface-treated pigments can be prepared according to surface treatment techniques of chemical, electronic, mechanochemical or mechanical nature which are well known to those skilled in the art. Commercial products can also be used.

The surface agent can be absorbed on, adsorbed on or grafted to the pigments by solvent evaporation, chemical reaction and creation of a covalent bond.

According to one variant, the surface treatment is constituted of a coating of the pigments.

The coating can represent from 0.1% to 20% by weight and in particular from 0.5% to 5% by weight of the total weight of the coated pigment.

The coating can be produced, for example, by adsorption of a liquid surface agent at the surface of the solid particles by simple mixing with stirring of the particles and of said surface agent, optionally with heating, prior to the incorporation of the particles in the other ingredients of the makeup or care composition.

The coating may be produced, for example, by chemical reaction of a surface agent with the surface of the solid pigment particles and creation of a covalent bond between the surface agent and the particles. This method is notably described in patent US 4 578 266.

The chemical surface treatment can consist in diluting the surface agent in a volatile solvent, in dispersing the pigments in this mixture and in then slowly evaporating the volatile solvent, so that the surface agent is deposited at the surface of the pigments.

When the pigment comprises a lipophilic or hydrophobic coating, the latter is preferably present in the fatty phase of the composition according to the invention.

According to a particular embodiment of the invention, the pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

According to a specific embodiment of the invention, the pigments can be coated by a hydrophilic compound.

According to a specific embodiment, the colorant is an organic pigment, which is synthetic, natural or of natural origin.

The term "organic pigment" is understood to mean any pigment which satisfies the definition of Ullmann's Encyclopedia in the chapter "Pigments, Organic". The organic pigment can in particular be chosen from nitroso, nitro, azo, xanthene, quinoline, anthraquinone, phthalocyanine, metal complex type, isoindolinone, isoindoline, quinacridone, perinone, perylene, diketopyrrolopyrrole, thioindigo, dioxazine, triphenylmethane or quinophthalone compounds.

The organic pigment(s) may be chosen, for example, from carmine, carbon black, aniline black, melanin, azo yellow, quinacridone, phthalocyanine blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenol derivatives as described in patent FR 2 679 771.

The pigments may also be in the form of composite pigments as described in patent EP 1 184 426. These composite pigments may notably be composed of particles including an inorganic core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The inorganic substrates onto which the dyes are adsorbed are, for example, alumina, silica, calcium sodium borosilicate or calcium aluminum borosilicate, and aluminum.

Mention may be made, among the organic dyes, of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 77 002), D&C Green 3 (CI 42 053), D&C Blue 1 (CI 42 090).

An example of a lake that may be mentioned is the product known under the name D&C Red 7 (CI 15 850:1).

The pigment(s) are preferably present in the composition in contents ranging from 1% to 30% by weight; and even better still from 3% to 25% by weight, relative to the total weight of the composition.

According to a particular embodiment of the invention, the colorant is a water-soluble dye or a liposoluble dye.

For the purposes of the invention, the term "water-soluble colorant" means any natural or synthetic, generally organic compound, which is soluble in an aqueous phase or water-miscible solvents and which is capable of imparting color.

For the purposes of the invention, the term "liposoluble colorant" means any natural or synthetic, generally organic compound, which is soluble in an oily phase or in solvents that are miscible with the oily phase, and which is capable of imparting color.

Mention may in particular be made, as water-soluble dyes which are suitable for the invention, of synthetic or natural water-soluble dyes, such as, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5 or FDC Blue 1.

Among the natural water-soluble dyes, mention may be made of anthocyanins.

As liposoluble dyes that are suitable for use in the invention, mention may notably be made, for instance, of the liposoluble dyes DC Red 17, DC Red 21, DC Red 27, DC Green 6, DC Yellow 11, DC Violet 2, DC Orange 5, Sudan red and Sudan brown.

As illustrations of natural liposoluble dyes, mention may be made particularly of carotenes, for instance $\beta$-carotene, -carotene and lycopene; quinoline yellow; xanthophylls such as astaxanthin, antheraxanthin, citranaxanthin, cryptoxanthin, canthaxanthin, diatomoxanthin, flavoxanthin, fucoxanthin, lutein, rhodoxanthin, rubixanthin, siphonaxanthin, violaxanthin, zeaxanthin; annatto; curcumin; quinizarin (Ceres Green BB, D&C Green No. 6, CI 61565, 1,4-di-p-toluidinoanthraquinone, Green No. 202, Quinazine Green SS) and chlorophylls.

The water-soluble or liposoluble dye(s) are preferably present in contents of less than 4% by weight, or even less than 2% by weight, more preferentially ranging from 0.01% to 2% by weight and even better still from 0.02% to 1.5% by weight, relative to the total weight of the composition.

Preferentially, the composition of the invention contains at least one pigment of metal oxide type, in particular chosen from iron oxides, preferably black iron oxides (CI 77499).

Preferably, said colorant is present in the composition in a content ranging from 1.0% to 30.0% by weight, preferably from 3.0% to 25.0% by weight, more particularly from 4.0% to 15.0% by weight, relative to the total weight of the composition.

### e) Cosmetic active agents

As cosmetic active agents that may be used in the compositions according to the invention, mention may be made notably of antioxidants, preserving agents, fragrances, neutralizers, cosmetic active agents, for instance emollients, vitamins and screening agents, in particular sunscreens, and mixtures thereof.

These additives can be present in the composition in a content ranging from 0.01% to 15.0% of the total weight of the composition.

Needless to say, those skilled in the art will take care to select the optional additional additives and/or the amount thereof such that the advantageous properties of the composition according to the invention are not, or are not substantially, adversely affected by the envisaged addition.

### f) Film-forming polymer

The compositions according to the present patent application can also comprise at least one hydrophilic or lipophilic, preferably hydrophilic, film-forming polymer.

In the present patent application, the term "film-forming polymer" means a polymer that is capable, by itself or in the presence of an auxiliary film-forming agent, of forming a macroscopically continuous deposit, and preferably a cohesive deposit, and even better still a deposit of which the cohesion and mechanical properties are such that said deposit can be isolated and manipulated individually, for example when said deposit is prepared by pouring onto a non-stick surface such as a Teflon-coated or silicone-coated surface.

In general, the content of "film-forming polymer" solids in the compositions according to the present patent application ranges from 0.1% to 30%, preferably from 0.5% to 20% and better still from 0.5% to 10% by weight relative to the total weight of the composition.

The hydrophilic film-forming polymer may be a water-soluble polymer or may be in dispersion in an aqueous medium.

Among the film-forming polymers that may be used in the composition of the present invention, mention may be made of synthetic polymers, of free-radical type or of polycondensate type, polymers of natural origin, and mixtures thereof.

As examples of water-soluble film-forming polymers, mention may be made of:

- proteins, for instance proteins of plant origin, such as wheat proteins, soya proteins;

- cellulose polymers such as hydroxyethylcellulose, hydroxypropylcellulose, methylcellulose, ethylhydroxyethylcellulose and carboxymethylcellulose, and also quaternized cellulose derivatives;

- acrylic polymers or copolymers, such as polyacrylates or polymethacrylates;

- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;

- gum arabic, guar gum, xanthan derivatives, karaya gum, acacia gum;

- alginates and carrageenans;

- glycosaminoglycans, hyaluronic acid and derivatives thereof;

- shellac resin, sandarac gum, dammar resins, elemi gums and copal resins;

- deoxyribonucleic acid;

- mucopolysaccharides such as chondroitin sulfates;

- and mixtures thereof.

The film-forming polymer may also be present in the composition in the form of particles dispersed in an aqueous phase, which is generally known as a latex or pseudolatex. Techniques for preparing these dispersions are well known to those skilled in the art.

As an aqueous dispersion of film-forming polymer, use may be made of the acrylic dispersions sold under the names Neocryl XK-90®, Neocryl A-1070®, Neocryl A-1090®, Neocryl BT-62®, Neocryl A-1079® and Neocryl A-523® by Avecia-Neoresins, Dow Latex 432® by Dow Chemical, Daitosol 5000 AD® or Daitosol 5000 SJ® by Daito Kasey Kogyo; Syntran 5760® by Interpolymer Allianz Opt®, by Rohm and Haas, or else the aqueous dispersions of polyurethane which are sold under the names Neorez R-981® and Neorez R-974® by Avecia-Neoresins, Avalure UR-405®, Avalure UR-410®, Avalure UR-425®, Avalure UR-450®, Sancure 875®, Avalure UR-445® and Sancure 2060® by Noveon, Impranil 85® by Bayer, Aquamere H-1511® by Hydromer; the sulfopolyesters sold under the brand name Eastman AQ® by Eastman Chemical Products, vinyl dispersions, for instance Mexomere PAM®, aqueous dispersions of polyvinyl acetate, for instance Vinybran® from Nisshin Chemical or those sold by Union Carbide, aqueous dispersions of terpolymer of vinylpyrrolidone, dimethylaminopropylmethacrylamide and lauryldimethylpropylmethacrylamidoammonium chloride, such as Styleze W from ISP, aqueous dispersions of polyurethane/polyacrylic hybrid polymers, such as those sold under the references Hybridur® by Air Products or Duromer® from National Starch, core/shell-type dispersions: for example, those sold by Atofina under the reference Kynar (core: fluoro- shell: acrylic) or else those described in document US 5 188 899 (core: silica - shell: silicone), and mixtures thereof.

A composition according to the invention may also comprise, as a variant of or additionally, a lipophilic polymer which may be in solution or as a dispersion in a non-aqueous solvent phase.

Preferentially, the composition of the invention comprises at least one hydrophilic film-forming polymer chosen from vinylpyrrolidone homopolymers.

According to a first particular form of the invention, the composition comprises, in particular in a physiologically acceptable medium:

(1) distearyl ether; and

(2) at least one anionic surfactant chosen from fatty acids having from 14 to less than 20 carbon atoms neutralized by (3) a cationic counterion chosen from amines and aminoalcohols , in particular the salt of stearic acid and of aminomethylpropanediol; and

(4) at least one fatty alcohol as defined above, preferentially cetyl alcohol; and

(5) water.

According to a second particular form of the invention, the composition comprises, in particular in a physiologically acceptable medium:

(1) distearyl ether; and

(2) at least one anionic surfactant chosen from $C_{14}$-$C_{22}$ acylglutamates neutralized by (3) an alkali metal cationic counterion, in particular the sodium salt of stearoyl glutamate; and

(4) at least one fatty alcohol as defined above, preferentially cetyl alcohol; and

(5) water.

According to a third particular form of the invention, the composition comprises, notably in a physiologically acceptable medium:

(1) distearyl ether; and

(2) at least one anionic surfactant chosen from $C_{12}$-$C_{22}$ monoalkyl phosphates neutralized by (3) an alkali metal cationic counterion, in particular the sodium salt of cetyl phosphate; and

(4) at least one fatty alcohol as defined above, preferentially cetyl alcohol; and

(5) water.
More preferentially, each of these particular forms of composition also comprises

(6) at least one wax as defined above, preferentially chosen from carnauba wax, beeswax and mixtures thereof; and

(7) at least one hydrophilic film-forming polymer as defined above, preferentially chosen from vinylpyrrolidone homopolymers.

## Physical characteristics

### a) Solids content

The composition according to the invention advantageously comprises a solids content of at least 38.0% by weight relative to the total weight of the composition, or even greater than 39% and less than 70% by weight, or even less than 60% by weight, or even less than 55% by weight, relative to the total weight of the composition.
For the purposes of the present invention, the term "solids content" denotes the content of nonvolatile matter.
The amount of solids content (abbreviated as SC) of a composition according to the invention is measured using a Halogen Moisture Analyzer HR 73® commercial halogen desiccator from Mettler Toledo. The measurement is performed on the basis of the weight loss of a sample dried by halogen heating, and thus represents the percentage of residual matter once the water and the volatile matter have evaporated off.
This technique is fully described in the machine documentation supplied by Mettler Toledo®.
The measuring protocol is as follows:
Approximately 2 g of the composition, referred to hereinbelow as the sample, are spread out on a metal crucible, which is placed in the halogen desiccator mentioned above. The sample is then subjected to a temperature of 105° C until a constant weight is obtained. The wet weight of the sample, corresponding to its initial weight, and the dry weight of the sample, corresponding to its weight after halogen heating, are measured using a precision balance.
The experimental error associated with the measurement is of the order of plus or minus 2%.
The solids content is calculated in the following manner:

[Math 1]

$$Solids\ Content\ (expressed\ in\ \%\ by\ weight) = 100 \times \frac{Dry\ weight}{Wet\ weight}$$

**b) Viscosity**

A composition according to the invention is advantageously creamy at an ambient temperature of 25° C.

It is characterized by a viscosity ranging from 5 to 50 Pa.s, measured at the ambient temperature of 25° C by means of a Rheomat RM100® rheometer, with a spindle 4 and with a shear rate of 200 rpm and a shear time of 10 min.

Such a viscosity is particularly advantageous since it is the most suitable for the device for applying mascara and since it enables easy use for the consumer for a charging result.

The composition may be manufactured via the known processes generally used in the cosmetics field.

**c) Suppleness**

A composition according to the invention has, at 25° C, a suppleness characterized by a hardness ≥ 20 g and preferably ranging from 30 g to 150 g.

The mascara texture is measured according to the following protocol:

The measuring device is a TA-XT-Plus sold by Swantech, equipped with a cell for measuring a force of 5 kg and with a cylindrical spindle 12.7 mm (1/2 inch) in diameter in Delrin. The mascara is thermostated at 20° C. It is then placed in excess in a container 60 mm in diameter and 22 mm deep using a metal spatula. The product is spread out in order to avoid any air pockets but without triturating it, so as not to destructure it. The container is then levelled off using a spatula so as to have a surface that is as uniform as possible. The container is then covered with a watch glass so as to limit the evaporation of solvents present in the formula for about 10 minutes. The options selected for this measuring method are the following:

Test mode: Compression measurement

Trigger force: 2.0 g

Pre-speed: 0.5 mm/sec

Test speed: 0.5 mm/sec

Temperature: 20° C +/- 1° C

Penetration distance: 5 mm.

Three successive measurements are carried out at points at least 12 mm apart, at least 10 mm from the edge of the container. The container is held in place during the measurement. The value selected is the mean of the maxima obtained at each measurement. The compositions according to the invention have penetrometry values of between 20 and 200 g, preferably between 30 and 150 g.

**d) Stability with respect to temperature variations**

The composition is monitored in extreme temperature conditions. The mascara is placed in a 100 ml glass container, then is placed in an oven with a temperature cycle ranging from 4 to 45° C, for 1 week according to the following program:

1 hour at 20° C

then 11 hours at 4° C

and finally 12 hours at 45° C.

The change from 4 to 45° C takes place over the course of approximately 20 to 30 min. On leaving the oven, the viscosity and the suppleness of the mascara are again evaluated. The "cycle" values are then compared with those performed at T24 h in order to decide on the stability of the formula suppleness. The compositions according to the invention have a suppleness variation with a value of between -10 g and +10 g of penetrometry.

The composition used according to the invention may be a makeup composition, a makeup base, notably for keratin fibers, or base coat, a composition to be applied onto makeup, also known as topcoat, or else a composition for treating keratin fibers.

More especially, the composition according to the invention is a mascara.

Such compositions are notably prepared according to the general knowledge of those skilled in the art.

## Packaging and application assembly or kit

The present invention also relates to an assembly, or kit, for packaging and applying a cosmetic composition for coating keratin fibers, comprising:

- a packaging device comprising a composition as described above,

- an applicator for said composition.

Said applicator may be integrally attached to a gripping member forming a cap for said packaging device. In other words, said applicator may be mounted in a removable position on said device between a closed position and an open position of a dispensing aperture of the device for packaging said composition.

An assembly for coating keratin fibers suitable for the invention may comprise an applicator configured for applying said cosmetic composition for coating keratin fibers and, where appropriate, a packaging device suitable for receiving said composition.

## Applicator

The applicator comprises means for smoothing and/or separating keratin fibers, such as the eyelashes or the eyebrows, notably in the form of teeth, bristles, spikes or other reliefs.

The applicator is arranged to apply the composition to the eyelashes or the eyebrows, and may comprise, for example, a brush or a comb.

The applicator may also be used for finishing of the makeup, over a region of the eyelashes or eyebrows that is made up or laden with the composition.

The brush may comprise a twisted core and bristles held between the turns of the core, or may be made in yet another way.

The comb is, for example, produced from a single part by molding of a plastic.

In certain exemplary embodiments, the application member is mounted at the end of a wand, which wand may be flexible, which may contribute to improving the comfort during application.

## Packaging device

The packaging device comprises a container intended for housing the composition for coating keratin fibers. This composition may then be withdrawn from the container by immersing the applicator therein.

This applicator may be firmly attached to a member for closing the container. This closing member may form a member for gripping the applicator. This gripping member may form a cap to be removably mounted on said container by any suitable means, such as by screwing, click-fastening, coupling, etc. Such a container may thus reversibly house said applicator.

This container may be optionally equipped with a wiper suitable for removing surplus product taken up by the applicator.

A process for applying the composition according to the invention to the eyelashes or the eyebrows may also include the following steps:

- forming a deposit of the cosmetic composition on the eyelashes or the eyebrows,

- leaving the deposit on the eyelashes or the eyebrows, it being possible for the deposit to dry.

It should be noted that, according to another embodiment, the applicator may form a product container. In such a case, a container may, for example, be provided in the gripping member and an internal channel can internally connect this gripping member to the application members in relief.

Finally, it should be noted that the packaging and application assembly may be in the form of a kit, it being possible for the applicator and the packaging device to be housed separately in the same packaging article.

The expressions "between ... and ..." and "ranging from ... to ..." should be understood as meaning limits included, unless otherwise specified.

In the description and the examples, the percentages are weight percentages, unless otherwise indicated. The percentages are thus expressed by weight, with respect to the total weight of the composition. The ingredients are mixed in the order and under the conditions which are readily determined by those skilled in the art.

The invention will now be described by means of examples which are present for purely illustrative purposes and should not be interpreted as examples that limit the invention.

### Examples 1 and 4 (invention) and examples C1, C2, C3 and C4 (outside the invention)

The following mascara examples (invention) and mascara examples C1, C2, C3 and C4 (outside the invention) were prepared.

[Table 1]

| Phase | Ingredients | Ex. C1 | Ex. 1 | Ex. C2 | Ex. 2 | Ex. C3 | Ex. C4 | Ex. 3 | Ex. 4 |
|---|---|---|---|---|---|---|---|---|---|
| A | POTASSIUM CETYL PHOSPHATE (AMPHISOL K®-DSM NUTRITIONAL PRODUCTS) | 5.0 | 5.0 | 5.0 | 5.0 | 5.0 | 5.0 | - | - |
| | SODIUM STEAROYL GLUTAMATE (AMISOFT HS 11 PF® - AJINOMOTO) | - | - | - | - | - | - | 5.0 | - |
| | STEARIC ACID (STEARINE TP 1200 PASTILLES® - STEARINERIE DUBOIS) | - | - | - | - | - | - | - | 5.0 |
| A | CETYL ALCOHOL (LANETTE 16®-BASF) | 7.0 | 7.0 | 7.0 | 7.0 | 7.0 | 7.0 | 7.0 | 7.0 |
| | BEHENYL BEHENATE (C44) (KESTER WAX K-72®- KOSTER KEUNEN) | 15.0 | - | 10.0 | - | - | - | - | - |
| | DISTEARYL ETHER (C36) (CUTINA STE®-BASF) | - | 15.0 | - | 10.0 | - | - | 10.0 | 10.0 |
| | MYRISTYL MYRISTATE (C28) (TEGOSOFT MM®-EVONIK GOLDSCHMIDT) | - | - | - | - | 10.0 | - | - | - |
| | ISOCETYL ISOSTEARATE (C34) (STEARATE D ISOCETYLE MB® (DUB SIS16®)-STEARINERIE DUBOIS) | - | - | - | - | - | 10.0 | - | - |
| | COPERNICIA CERIFERA (CARNAUBA) WAX | - | - | 2.9 | 2.9 | 2.9 | 2.9 | 2.9 | 2.9 |
| | BEESWAX | - | - | 3.6 | 3.6 | 3.6 | 3.6 | 3.6 | 3.6 |
| | BLACK IRON OXIDES/CI 77499 | 10.0 | 10.0 | 10.0 | 10.0 | 10.0 | 10.0 | 10.0 | 10.0 |

(continued)

| Phase | Ingredients | Ex. C1 | Ex. 1 | Ex. C2 | Ex. 2 | Ex. C3 | Ex. C4 | Ex. 3 | Ex. 4 |
|---|---|---|---|---|---|---|---|---|---|
| B | AMINOMETHYL PROPANEDIOL | 0.2 | 0.2 | - | | - | - | - | 0.7 |
| | POLYVINYLPYRROL IDONE (LUVISKOL K 30 POUDRE®- BASF) | 3 | 3 | - | | - | - | - | - |
| | PHENOXYETHANOL | 0.5 | 0.5 | 0.5 | 0.5 | 0.5 | 0.5 | 0.5 | 0.5 |
| | PHENETHYL ALCOHOL (130744 PHENYLETHYL ALCOHOL® - SYMRISE) | 0.5 | 0.5 | 0.5 | 0.5 | 0.5 | 0.5 | 0.5 | 0.5 |
| | Water | qs 100 | qs 100 | qs 100 | qs 100 | qs 100 | qs 100 | qs 100 | qs 100 |

## Protocol for preparing examples 1 to 4 (invention) and examples C1, C2, C3 and C4 (outside the invention)

### Preparation of phase A

The starting materials were carefully weighed out beforehand using a balance (precision = 0.01 g). The ingredients of phase A were melted in a jacketed heating pan in which circulates an oil of which the temperature is controlled by means of a thermostatically-controlled oil bath. The setpoint temperature was set at 90° C. After total melting, the pigment was introduced with stirring using a Rayneri® blender. Stirring was maintained until a homogeneous preparation was obtained.

### Preparation of phase B

The water was preheated to 95° C in an electric kettle. The ingredients of phase B were mixed with the hot water and then the preserving agents were introduced into the water in a beaker at a temperature of 80° C with stirring using a Rayneri® blender.

### Emulsification of phases A and B

Phase B was poured into phase A with stirring for 5 minutes at 90° C using a Rayneri blender. Phase A + B was then cooled to ambient temperature with stirring.

### Packaging of the formulation

The mascara formula obtained was then transferred into a hermetically closed jar in order to prevent drying of the formula in contact with the air.

### Comparative tests

The mascara was first observed macroscopically, with the naked eye, to estimate its texture according to the following scale:

Fluid

Supple

Thick

Compact.

Its roughness was also observed: smooth/not smooth.
Its transfer capacity was also observed: deposits well/light deposit/does not deposit.
Its stability was also determined visually after a few days: stable/unstable.

The results obtained are shown in table 2 below.

[Table 2]

| Ingredients | Ex. C1 | Ex. 1 | Ex. C2 | Ex. 2 | Ex. C3 | Ex. C4 | Ex. 3 | Ex. 4 |
|---|---|---|---|---|---|---|---|---|
| **Appearance of the texture** | Very supple and unstable cream | Thick, smooth and stable cream | Compact cream which does not deposit | Thick, smooth and stable cream | Compact cream which does not deposit | Cream too fluid with light deposit | Fluid, supple, smooth and stable cream | Thick, smooth and stable cream |
| **Viscosity Rheomat RM100® spindle 4, speed 200 rpm for 10 min of shear** | 10.6 Pa. s | 32.8 Pa.s | 34.2 Pa.s | 18.9 Pa.s | 39.4 Pa.s | 1.2 Pa.s | 5 Pa.s | 27.9 Pa.s |
| **Suppleness** | 32 g | 79 g | 168 g | 76 g | 325 g | 72 g | 20 g | 95 g |
| **Suppleness var-iation at the tem-perature cycle described above** | +71 g | +7 g | -17 g | -3 g | -38 g | +4 g | +7 g | 10 g |

It was observed that examples 1 to 4 according to the invention comprising a fatty dialkyl ether of formula (I) (distearyl ether having 36 carbon atoms) exhibited a stable texture at the temperature cycle described above, namely creamy, supple, smooth and sufficiently thick for good application to the eyelashes, unlike examples C1, C2, C3 and C4 outside the invention comprising, in place of the dialkyl ether, an ester of a fatty alcohol and of a fatty acid, having 28, 34 or 44 carbon atoms.

## Claims

1. A composition comprising, notably in a physiologically acceptable medium:

   (1) at least one fatty dialkyl ether of formula (I) below:

   $$R_1\text{-}O\text{-}R_2 \qquad (I)$$

   wherein R1 and R2, which may be identical or different, denote a linear and saturated alkyl radical having a number of carbon atoms ranging from 16 to 20; and
   (2) at least one anionic surfactant comprising (3) at least one cationic counterion, preferably at least one anionic surfactant in a form partially or totally neutralized by said cationic counterion and

   (4) at least one nonionic surfactant with an HLB of less than or equal to 8; and
   (5) water.

2. The composition as claimed in claim 1, wherein the fatty dialkyl ether(s) of formula (I) are present in a content of at least 3% by weight, preferably in a content of at least 5% by weight, more preferentially in a content ranging from 5.0% to 35.0% by weight, or even more preferentially in a content from 8.0% to 25.0% by weight, relative to the total weight of the composition.

3. The composition as claimed in claim 1 or 2, wherein the fatty dialkyl ether of formula (I) is chosen from:

   - the dicetyl ether having the structure:

   $$CH_3(CH_2)_{15}O(CH_2)_{15}CH_3$$

- the distearyl ether having the structure:

$$CH_3(CH_2)_{17}O(CH_2)_{17}CH_3$$

mixtures thereof, and more particularly is distearyl ether.

4. The composition as claimed in any one of the preceding claims, wherein the anionic surfactant(s) have an HLB of greater than or equal to 8.

5. The composition as claimed in any one of the preceding claims, wherein the anionic surfactant(s) is (are) chosen from:

    i. $C_{12}$-$C_{20}$ monoalkyl phosphates;
    ii. alkyl sulfates, and in particular alkyl ether sulfates, alkylamido ether sulfates, alkylaryl polyether sulfates, monoglyceride sulfates;
    iii. alkylsulfonates, alkylamidesulfonates, alkylarylsulfonates, $\alpha$-olefinsulfonates, paraffinsulfonates;
    iv. alkyl sulfosuccinates, alkyl ether sulfosuccinates, alkylamide sulfosuccinates; alkyl sulfosuccinamates;
    v. acyl sarcosinates, acyl glutamates, acyl isethionates, N-acyl taurates and acyl lactylates;
    vi. fatty acids, in particular palmitic acid or stearic acid, coconut oil acid or hydrogenated coconut oil acid;
    vii mixtures thereof.

6. The composition as claimed in any one of the preceding claims, wherein the cationic counterion(s) is (are) chosen from a cation of mineral origin, in particular chosen from alkali metal cations, alkaline-earth metal cations, the ammonium ion ($NH_4^+$) or organic cations, notably chosen from amines and aminoalcohols, in particular a primary (poly) hydroxyalkylamine, more particularly 1,3-dihydroxy-2 methylpropyl.

7. The composition as claimed in any one of the preceding claims, wherein the anionic surfactant(s) is (are) present in a total content of greater than or equal to 2% by weight relative to the total weight of the composition, preferably ranging from 3% to 15%, better still from 3% to 12% by weight, relative to the total weight of the composition.

8. The composition as claimed in any one of the preceding claims, wherein the anionic surfactant(s) is (are) chosen from:

    a) $C_{12}$-$C_{22}$ monoalkyl phosphates;
    b) $C_{12}$-$C_{22}$ acylglutamates;
    c) fatty acids containing from 14 to less than 20 carbon atoms;
    d) and mixtures thereof.

9. The composition as claimed in any one of the preceding claims, wherein the anionic surfactant is the potassium salt of monocetyl phosphate, having the INCI name: Potassium Cetyl Phosphate.

10. The composition as claimed in any one of the preceding claims, comprising at least 30.0% by weight of water, better still at least 40.0% by weight, more particularly at least 45% by weight, and even more particularly a content of from 45% to 65% by weight of water, relative to the total weight of the composition.

11. The composition as claimed in any one of the preceding claims, also comprising at least one wax, preferably chosen from carnauba wax, beeswax and mixtures thereof.

12. The composition as claimed in claim 11, wherein the wax(es) are present in a content of less than 30.0%, preferably less than 20.0%, notably ranging from 2% to 18% by weight of wax, more preferentially from 3% to 15% by weight, relative to the total weight of the composition.

13. The composition as claimed in any one of the preceding claims, wherein the nonionic surfactant(s) with an HLB value of greater than or equal to 8 at 25° C is (are) chosen from:

    - fatty alcohols, notably $C_{14}$-$C_{30}$, and more preferentially $C_{14}$-$C_{24}$, more particularly $C_{14}$-$C_{20}$, fatty alcohols;
    - ethers of a fatty alcohol, notably a $C_8$-$C_{24}$ fatty alcohol and of polyethylene glycol (PEG) comprising from 1 to 10 ethylene glycol units, preferably from 2 to 10 ethylene glycol units;
    - esters of fatty acids, notably $C_8$-$C_{24}$ and preferably of $C_{16}$-$C_{22}$ fatty acids, and of glycerol;

and mixtures thereof.

14. The composition as claimed in any one of the preceding claims, wherein the nonionic surfactant(s) are chosen from fatty alcohols with an HLB of less than or equal to 8 at 25° C, notably chosen from cetyl alcohol, stearyl alcohol and mixtures thereof; and more particularly cetyl alcohol.

15. The composition as claimed in claim 14, wherein the fatty alcohol nonionic surfactant(s) is (are) present in amounts ranging from 1.0% to 10.0% by weight, relative to the total weight of the composition, preferably from 2.0% to 10% by weight, and even more preferentially from 3% to 8% by weight, relative to the total weight of the composition.

16. The composition as claimed in any one of claims 1 to 13, wherein the nonionic surfactants) are chosen from ethers of a fatty alcohol, notably a $C_8$-$C_{24}$ fatty alcohol, and of polyethylene glycol (PEG) comprising from 1 to 10 ethylene glycol units, and more particularly is Steareth-2.

17. The composition as claimed in claim 16, wherein the nonionic surfactant(s) which is an (are) ether(s) of a fatty alcohol and of polyethylene glycol with an HLB value at 25° C of less than or equal to 8 is (are) present at from 1.0% to 10% by weight, relative to the total weight of the composition, preferably from 2.0% to 10% by weight, and even more particularly from 3% to 8% by weight, relative to the total weight of the composition.

18. The composition as claimed in any one of claims 1 to 13, wherein the nonionic surfactant(s) are chosen from esters of fatty acids, notably $C_8$-$C_{24}$ and preferably $C_{16}$-$C_{22}$ fatty acids, and of glycerol, and more particularly is glyceryl monostearate.

19. The composition as claimed in claim 18, wherein the nonionic surfactant(s) which is an (are) ester(s) of a fatty acid, notably a $C_8$-$C_{24}$ and preferably $C_{16}$-$C_{22}$ fatty acid, and of glycerol with an HLB value at 25° C of less than or equal to 8 is (are) present in a content of from 1.0% to 10% by weight, relative to the total weight of the composition, preferably from 2.0% to 10% by weight, and even more particularly from 3% to 8% by weight, relative to the total weight of the composition.

20. The composition as claimed in any one of the preceding claims, also comprising at least one liquid fatty phase.

21. The composition as claimed in claim 20, comprising at least one nonvolatile oil, chosen notably from hydrocarbon-based oils.

22. The composition as claimed in claim 20 or 21, comprising less than 2.0% by weight of volatile oil(s), or even less than 1.0% relative to the total weight of the composition; more particularly, said composition is free of volatile oil.

23. The composition as claimed in any one of the preceding claims, also comprising at least one colorant, notably chosen from pigments, liposoluble dyes, water-soluble dyes and mixtures thereof; more preferentially chosen from metal oxides; more particularly chosen from iron oxides, and even more particularly chosen from black iron oxides (CI 77499).

24. The composition as claimed in claim 23, wherein the colorant(s) is (are) present in a content ranging from 1.0% to 30.0% by weight, preferably from 3.0% to 25.0% by weight, more particularly from 4.0% to 15.0% by weight, relative to the total weight of the composition.

25. The composition as claimed in any one of the preceding claims, also comprising at least one hydrophobic or lipophilic, preferably hydrophilic, film-forming polymer, and more particularly a vinylpyrrolidone homopolymer.

26. The composition as claimed in any one of the preceding claims, in the form of an eyelash product such as a mascara, an eyebrow product or a product for the area around the eyes, such as an eyeliner.

27. An assembly, or kit, for packaging and applying a cosmetic composition for coating keratin fibers, comprising:

- a packaging device comprising the composition as defined in any one of the preceding claims;
- an applicator for said composition.

28. A process for coating keratin materials, in particular the skin, notably the area around the eyes or the contour of the eyelashes or eyebrows, and keratin fibers, such as the eyelashes and/or the eyebrows, comprising a step of applying to said keratin materials at least one composition as defined in any one of claims 1 to 26.

**Patentansprüche**

1.  Zusammensetzung, umfassend, insbesondere in einem physiologisch unbedenklichen Medium:

    (1) mindestens einen Fettdialkylether der folgenden Formel (I):

    $$R_1\text{-}O\text{-}R_2 \qquad (I)$$

    wobei R1 und R2, die gleich oder verschieden sein können, einen linearen und gesättigten Alkylrest mit einer Zahl von Kohlenstoffatomen im Bereich von 16 bis 20 bedeuten; und
    (2) mindestens ein anionisches Tensid, umfassend (3) mindestens ein kationisches Gegenion, vorzugsweise mindestens ein anionisches Tensid in einer durch das kationische Gegenion teilweise oder vollständig neutralisierten Form, und
    (4) mindestens ein nichtionisches Tensid mit einem HLB-Wert kleiner oder gleich 8 und
    (5) Wasser.

2.  Zusammensetzung nach Anspruch 1, wobei der Fettdialkylether bzw. die Fettdialkylether der Formel (I) in einem Gehalt von mindestens 3 Gew.-%, vorzugsweise in einem Gehalt von mindestens 5 Gew.-%, weiter bevorzugt in einem Gehalt von 5,0 Gew.-% bis 35,0 Gew.-% oder noch weiter bevorzugt in einem Gehalt von 8,0 Gew.-% bis 25,0 Gew.-%, bezogen auf das Gesamtgewicht der Zusammensetzung, vorliegt bzw. vorliegen.

3.  Zusammensetzung nach Anspruch 1 oder 2, wobei der Fettdialkylether der Formel (I) aus

    - dem Dicetylether mit der folgenden Struktur:

    $$CH_3(CH_2)_{15}O(CH_2)_{15}CH_3$$

    - dem Distearylether mit der folgenden Struktur:

    $$CH_3(CH_2)_{17}O(CH_2)_{17}CH_3;$$

    Mischungen davon ausgewählt ist und spezieller Distearylether ist.

4.  Zusammensetzung nach einem der vorhergehenden Ansprüche, wobei das anionische Tensid bzw. die anionischen Tenside einen HLB-Wert größer oder gleich 8 aufweist bzw. aufweisen.

5.  Zusammensetzung nach einem der vorhergehenden Ansprüche, wobei das anionische Tensid bzw. die anionischen Tensid ausgewählt sind aus:

    i. $C_{12}$-$C_{20}$-Monoalkylphosphaten;
    ii. Alkylsulfaten und insbesondere Alkylethersulfaten, Alkylamidoethersulfaten, Alkylarylpolyethersulfaten, Monoglyceridsulfaten;
    iii. Alkylsulfonaten, Alkylamidsulfonaten, Alkylarylsulfonaten, $\alpha$-Olefinsulfonaten, Paraffinsulfonaten;
    iv. Alkylsulfosuccinaten, Alkylethersulfosuccinaten, Alkylamidsulfosuccinaten; Alkylsulfosuccinamaten;
    v. Acylsarcosinaten, Acylglutamaten, Acylisethionaten, N-Acyltauraten und Acyllactylaten;
    vi. Fettsäuren, insbesondere Palmitinsäure oder Stearinsäure, Kokosölsäure oder hydrierter Kokosölsäure;
    vii. Mischungen davon.

6.  Zusammensetzung nach einem der vorhergehenden Ansprüche, wobei das kationische Gegenion bzw. die kationischen Gegenionen aus einem Kation mineralischen Ursprungs, insbesondere ausgewählt aus Alkalimetallkationen, Erdalkalimetallkationen, dem Ammoniumion ($NH_4^+$) oder organischen Kationen, insbesondere ausgewählt aus Aminen und Aminoalkoholen, insbesondere einem primären (Poly)hydroxyalkylamin, spezieller 1,3-Dihydroxy-2-methylpropyl, ausgewählt ist bzw. sind.

7. Zusammensetzung nach einem der vorhergehenden Ansprüche, wobei das anionische Tensid bzw. die anionischen Tenside in einem Gesamtgehalt größer oder gleich 2 Gew.-%, bezogen auf das Gesamtgewicht der Zusammensetzung, vorzugsweise im Bereich von 3 bis 15 Gew.-%, noch besser von 3 bis 12 Gew.-%, bezogen auf das Gesamtgewicht der Zusammensetzung, vorliegt bzw. vorliegen.

8. Zusammensetzung nach einem der vorhergehenden Ansprüche, wobei das anionische Tensid bzw. die anionischen Tensid ausgewählt sind aus:

   a) $C_{12}$-$C_{22}$-Monoalkylphosphaten;
   b) $C_{12}$-$C_{22}$-Acylglutamaten;
   c) Fettsäuren mit 14 bis weniger als 20 Kohlenstoffatomen;
   d) und Mischungen davon.

9. Zusammensetzung nach einem der vorhergehenden Ansprüche, wobei es sich bei dem anionischen Tensid um das Kaliumsalz von Monocetylphosphat mit der folgenden INCI-Bezeichnung handelt: Potassium Cetyl Phosphate.

10. Zusammensetzung nach einem der vorhergehenden Ansprüche, umfassend mindestens 30,0 Gew.-% Wasser, noch besser mindestens 40,0 Gew.-%, spezieller mindestens 45 Gew.-% und noch spezieller einen Gehalt von 45 Gew.-% bis 65 Gew.-% Wasser, bezogen auf das Gesamtgewicht der Zusammensetzung.

11. Zusammensetzung nach einem der vorhergehenden Ansprüche, ferner umfassend mindestens ein Wachs, vorzugsweise ausgewählt aus Carnaubawachs, Bienenwachs und Mischungen davon.

12. Zusammensetzung nach Anspruch 11, wobei das Wachs bzw. die Wachse in einem Gehalt von weniger als 30,0 Gew.-%, vorzugsweise weniger als 20,0 Gew.-%, insbesondere im Bereich von 2 bis 18 Gew.-%, Wachs, weiter bevorzugt von 3 bis 15 Gew.-%, bezogen auf das Gesamtgewicht der Zusammensetzung, vorliegt bzw. vorliegen.

13. Zusammensetzung nach einem der vorhergehenden Ansprüche, wobei das nichtionische Tensid bzw. die nichtionischen Tenside mit einem HLB-Wert größer oder gleich 8 bei 25 °C ausgewählt ist bzw. sind aus:

   - Fettalkoholen, insbesondere $C_{14}$-$C_{30}$- und weiter bevorzugt $C_{14}$-$C_{24}$-Fettalkoholen, spezieller $C_{14}$-$C_{20}$-Fettalkoholen;
   - Ethern eines Fettalkohols, insbesondere eines $C_8$-$C_{24}$-Fettalkohols, und von Polyethylenglykol (PEG) mit 1 bis 10 Ethylenglykoleinheiten, vorzugsweise 2 bis 10 Ethylenglykoleinheiten;
   - Estern von Fettsäuren, insbesondere von $C_8$-$C_{24}$- und vorzugsweise von $C_{16}$-$C_{22}$-Fettsäuren, und von Glycerin; und Mischungen davon.

14. Zusammensetzung nach einem der vorhergehenden Ansprüche, wobei das nichtionische Tensid bzw. die nichtionischen Tenside aus Fettalkoholen mit einem HLB-Wert kleiner oder gleich 8 bei 25 °C, insbesondere ausgewählt aus Cetylalkohol, Stearylalkohol und Mischungen davon und spezieller Cetylalkohol, ausgewählt ist bzw. sind.

15. Zusammensetzung nach Anspruch 14, **dadurch gekennzeichnet, dass** das nichtionische Fettalkoholtensid bzw. die nichtionischen Fettalkoholtenside in Mengen im Bereich von 1,0 bis 10,0 Gew.-%, bezogen auf das Gesamtgewicht der Zusammensetzung, vorzugsweise von 2,0 bis 10 Gew.-% und noch weiter bevorzugt von 3 bis 8 Gew.-%, bezogen auf das Gesamtgewicht der Zusammensetzung, vorliegt bzw. vorliegen.

16. Zusammensetzung nach einem der Ansprüche 1 bis 13, wobei das nichtionische Tensid bzw. die nichtionischen Tenside aus Ethern eines Fettalkohols, insbesondere eines $C_8$-$C_{24}$-Fettalkohols, und von Polyethylenglykol (PEG) mit 1 bis 10 Ethylenglykoleinheiten ausgewählt ist bzw. sind und es sich dabei spezieller um Steareth-2 handelt.

17. Zusammensetzung nach Anspruch 16, wobei das nichtionische Tensid bzw. die nichtionischen Tenside, bei dem bzw. denen es sich um einen oder mehrere Ether von einem Fettalkohol und von Polyethylenglykol mit einem HLB-Wert bei 25 °C kleiner oder gleich 8 handelt, in einem Gehalt von 1,0 bis 10 Gew.-%, bezogen auf das Gesamtgewicht der Zusammensetzung, vorzugsweise 2,0 bis 10 Gew.-% und noch spezieller 3 bis 8 Gew.-%, bezogen auf das Gesamtgewicht der Zusammensetzung, vorliegt bzw. vorliegen.

18. Zusammensetzung nach einem der Ansprüche 1 bis 13, wobei das oder die nichtionischen Tenside aus Estern von Fettsäuren, insbesondere $C_8$-$C_{24}$- und vorzugsweise $C_{16}$-$C_{22}$-Fettsäuren, und von Glycerin ausgewählt ist bzw. sind und es sich dabei spezieller um Glycerylmonostearat handelt.

19. Zusammensetzung nach Anspruch 18, wobei das nichtionisches Tensid bzw. die nichtionischen Tenside, bei denen es sich um einen oder mehrere Ester einer Fettsäure, insbesondere einer $C_8$-$C_{24}$- und vorzugsweise $C_{16}$-$C_{22}$-Fettsäure, und von Glycerin mit einem HLB-Wert bei 25 °C kleiner oder gleich 8 handelt, in einem Gehalt von 1,0 bis 10 Gew.-%, bezogen auf das Gesamtgewicht der Zusammensetzung, vorzugsweise von 2,0 bis 10 Gew.-%, und insbesondere 3 bis 8 Gew.-%, bezogen auf das Gesamtgewicht der Zusammensetzung, vorliegt bzw. vorliegen.

20. Zusammensetzung nach einem der vorhergehenden Ansprüche, die außerdem mindestens eine flüssige Fettphase umfasst.

21. Zusammensetzung nach Anspruch 20, umfassend mindestens ein nichtflüchtiges Öl, das insbesondere aus Ölen auf Kohlenwasserstoffbasis ausgewählt ist.

22. Zusammensetzung nach Anspruch 20 oder 21, umfassend weniger als 2,0 Gew.-% flüchtiges Öl bzw. flüssige Öle oder sogar weniger als 1,0 %, bezogen auf das Gesamtgewicht der Zusammensetzung; wobei die Zusammensetzung spezieller frei von flüchtigem Öl ist.

23. Zusammensetzung nach einem der vorhergehenden Ansprüche, außerdem umfassend mindestens ein Farbmittel, insbesondere ausgewählt aus Pigmenten, fettlöslichen Farbstoffen, wasserlöslichen Farbstoffen und Mischungen davon; weiter bevorzugt ausgewählt aus Metalloxiden; spezieller ausgewählt aus Eisenoxiden und noch spezieller aus schwarzen Eisenoxiden (CI 77499).

24. Zusammensetzung nach Anspruch 23, wobei das Farbmittel bzw. die Farbmittel in einem Gehalt im Bereich von 1,0 bis 30,0 Gew.-%, vorzugsweise von 3,0 bis 25,0 Gew.-%, spezieller von 4,0 bis 15,0 Gew.-%, bezogen auf das Gesamtgewicht der Zusammensetzung, vorliegt bzw. vorliegen.

25. Zusammensetzung nach einem der vorhergehenden Ansprüche, außerdem umfassend mindestens ein hydrophobes oder lipophiles, vorzugsweise hydrophiles, filmbildendes Polymer, insbesondere ein Vinylpyrrolidon-Homopolymer.

26. Zusammensetzung nach einem der vorhergehenden Ansprüche in Form eines Wimpernprodukts, wie etwa einer Mascara, eines Augenbrauenprodukts oder eines Produkts für den Bereich um die Augen herum, wie eines Eyeliners.

27. Anordnung oder Kit zum Verpacken und Aufbringen einer kosmetischen Zusammensetzung zum Beschichten von Keratinfasern, umfassend:

   - eine Verpackungsvorrichtung, die die Zusammensetzung gemäß einem der vorhergehenden Ansprüche umfasst;
   - einen Applikator für die Zusammensetzung.

28. Verfahren zum Beschichten von Keratinmaterialien, insbesondere der Haut, insbesondere des Bereichs um die Augen herum oder der Kontur der Wimpern oder Augenbrauen, und Keratinfasern, wie den Wimpern und/oder den Augenbrauen, umfassend einen Schritt des Aufbringens mindestens einer Zusammensetzung gemäß einem der Ansprüche 1 bis 26 auf die Keratinmaterialien.

**Revendications**

1. Composition comprenant, notamment dans un milieu physiologiquement acceptable :

   (1) au moins un dialkyl éther gras de formule (I) suivante :

   $$R_1\text{-O-}R_2 \qquad (I)$$

   dans laquelle R1 et R2, identiques ou différents, désignent un radical alkyle linéaire et saturé et possédant un nombre d'atomes de carbone allant de 16 à 20 ; et
   (2) au moins un tensioactif anionique comprenant (3) au moins un contre-ion cationique, de préférence au moins un tensioactif anionique sous forme partiellement ou totalement neutralisée par ledit contre-ion cationique et
(4) au moins un tensioactif non-ionique de HLB inférieur ou égal à 8 ; et
(5) de l'eau.

2. Composition selon la revendication 1, où le ou les dialkyl éther(s) gras de formule (I) sont présents dans une teneur d'au moins 3% en poids, de préférence dans une teneur d'au moins 5% en poids, plus préférentiellement dans une teneur variant de 5,0 à 35,0 % en poids, voire plus préférentiellement dans une teneur de 8,0 à 25,0 % en poids, par rapport au poids total de la composition.

3. Composition selon la revendication 1 ou 2, dans laquelle le dialkyl éther gras de formule (I) est choisi parmi

- le dicetylether de structure :

$$CH_3(CH_2)_{15}O(CH_2)_{15}CH_3$$

- le distearylether de structure :

$$CH_3(CH_2)_{17}O(CH_2)_{17}CH_3 \; ;$$

et leurs mélanges, et plus particulièrement est le distearyl ether.

4. Composition selon l'une quelconque des revendications précédentes, dans laquelle le ou les tensioactifs anioniques ont un HLB supérieur ou égal à 8.

5. Composition selon l'une quelconque des revendications précédentes, dans laquelle le ou les tensioactifs anioniques sont choisis parmi :

i. les phosphates de mono-alkyle en $C_{12}$ -$C_{20}$ ;
ii. les alkylsulfates, et en particulier les alkyléthersulfates, les alkylamidoéthersulfates, les alkylarylpolyéthersulfates, les monoglycérides sulfates ;
iii. les alkylsulfonates, alkylamidesulfonates, alkylarylsulfonates, $\alpha$-oléfine-sulfonates, paraffine-sulfonates ;
iv. les alkylsulfosuccinates, les alkyléthersulfosuccinates, les alkylamidesulfosuccinates ; les alkyl sulfosuccinamates ;
v. les acylsarcosinates, les acylglutamates, les acyliséthionates, les N-acyltaurates, les acyllactylates ;
vi. les acides gras, en particulier les acides palmitique, stéarique, les acides d'huile de coprah ou d'huile de coprah hydrogénée ;
vii. leurs mélanges.

6. Composition selon l'une quelconque des revendications précédentes, dans laquelle le ou les contre-ion(s) cationique(s) est (sont) choisi(s) parmi un cation d'origine minérale, en particulier choisi parmi les cations de métal alcalin, les cations de métal alcalino-terreux, l'ion ammonium ($NH_4^+$) ou les cations organiques, notamment choisis parmi les amines et les amino-alcools, en particulier un (poly)hydroxyalkylamine primaire, plus particulièrement le 1,3-dihydroxy-2 méthyle propyle.

7. Composition selon l'une quelconque des revendications précédentes, dans laquelle le ou les tensioactif(s) anionique(s) est (sont) présent(s) à une teneur totale supérieure ou égale à 2 % en poids par rapport au poids total de la composition, de préférence allant de 3 % à 15 %, mieux de 3 % à 12 % en poids, par rapport au poids total de la composition.

8. Composition selon l'une quelconque des revendications précédentes, dans laquelle le ou les tensioactif(s) anionique(s) est (sont) choisi(s) parmi :

a) les phosphates de mono-alkyle en $C_{12}$-$C_{22}$ ;
b) les acylglutamates en $C_{12}$-$C_{22}$ ;
c) les acides gras ayant de 14 à moins de 20 atomes de carbone ;
d) et leurs mélanges.

9. Composition selon l'une quelconque des revendications précédentes, dans laquelle le tensioactif anionique est le sel de potassium de phosphate de mono-cétyle, de nom INCI : Potassium Cetyl Phosphate.

10. Composition selon l'une quelconque des revendications précédentes, comprenant au moins 30,0 % en poids d'eau, mieux au moins 40,0 % en poids, plus particulièrement au moins 45% en poids, et encore plus particulièrement une teneur de 45 à 65 % en poids d'eau, par rapport au poids total de la composition.

11. Composition selon l'une quelconque des revendications précédentes, comprenant en plus au moins une cire, de préférence choisie parmi la cire de carnauba, la cire d'abeille, et leurs mélanges.

12. Composition selon la revendication 11, où la ou les cires sont présentes à une teneur inférieure à 30,0 %, de préférence inférieure à 20,0 %, notamment allant de 2 à 18 % en poids de cire, plus préférentiellement de 3 à 15 % en poids par rapport au poids total de la composition.

13. Composition selon l'une quelconque des revendications précédentes, où le (ou les) tensioactif(s) non ionique(s) de HLB supérieur ou égal à 8, à 25°C, est (sont) choisi(s) parmi :

   - les alcools gras, notamment en $C_{14}$-$C_{30}$, et plus préférentiellement en $C_{14}$-$C_{24}$, plus particulièrement en $C_{14}$-$C_{20}$ ;
   - les éthers d'alcool gras, notamment en $C_8$-$C_{24}$ et de polyéthylène glycol (PEG) comprenant de 1 à 10 motifs d'éthylèneglycol, de préférence de 2 à 10 motifs d'éthylèneglycol ;
   - les esters d'acides gras, notamment en $C_8$-$C_{24}$, et de préférence en $C_{16}$-$C_{22}$, et de glycérol ;

   et leurs mélanges.

14. Composition selon l'une quelconque des revendications précédentes, où le ou les tensioactif(s) non-ionique(s) sont choisis parmi les alcools gras de HLB inférieur ou égal à 8 à 25°C, notamment choisis parmi l'alcool cétylique, l'alcool stéarylique et leurs mélanges ; et plus particulièrement l'alcool cétylique.

15. Composition selon la revendication 14, où le ou les tensioactif(s) non-ionique(s) alcool(s) gras est (sont) présent(s) dans des quantités allant de 1,0 à 10,0 % en poids, par rapport au poids total de la composition, de préférence de 2,0 à 10 % en poids, et encore plus particulièrement de 3 à 8 % en poids, par rapport au poids total de la composition.

16. Composition selon l'une quelconque des revendications 1 à 13, où le ou les tensioactif(s) non-ionique(s) sont choisis parmi les éthers d'alcool gras, notamment en $C_8$-$C_{24}$ et de polyéthylène glycol (PEG) comprenant de 1 à 10 motifs d'éthylèneglycol, et plus particulièrement est le Steareth-2.

17. Composition selon la revendication 16, où le ou les tensioactif(s) non-ionique(s) éther(s) d'alcool gras et de polyéthylène glycol de valeur HLB à 25°C inférieure ou égale à 8, est (sont) présent (s) de 1,0 à 10,0 % en poids, par rapport au poids total de la composition, de préférence de 2,0 à 10 % en poids, et encore plus particulièrement de 3 à 8 % en poids, par rapport au poids total de la composition.

18. Composition selon l'une quelconque des revendications 1 à 13, où le ou les tensioactif(s) non-ionique(s) sont choisis parmi les esters d'acides gras, notamment en $C_8$-$C_{24}$, et de préférence en $C_{16}$-$C_{22}$, et de glycérol, et plus particulièrement est le monostéarate de glycéryle.

19. Composition selon la revendication 18, où le ou les tensioactif(s) non-ionique(s) ester(s) d'acide gras notamment, en $C_8$-$C_{24}$, et de préférence en $C_{16}$-$C_{22}$, et de glycérol de valeur HLB à 25°C inférieure ou égale à 8, est (sont) présent(s) à une teneur de 1,0 à 10,0 % en poids, par rapport au poids total de la composition, de préférence de 2,0 à 10 % en poids, et encore plus particulièrement de 3 à 8 % en poids, par rapport au poids total de la composition.

20. Composition selon l'une quelconque des revendications précédentes, comprenant en plus au moins une phase grasse liquide.

21. Composition selon la revendication 20, comprenant au moins une huile non volatile, notamment choisie parmi les huiles hydrocarbonées.

22. Composition selon la revendication 20 ou 21, comprenant moins de 2, 0% en poids d'huile(s) volatile(s), voire moins de 1,0% par rapport au poids total de la composition ; plus particulièrement, ladite composition est dépourvue d'huile volatile.

23. Composition selon l'une quelconque des revendications précédentes, comprenant en plus au moins une matière colorante, notamment choisie parmi les pigments, les colorants liposolubles, les colorants hydrosolubles et leurs mélanges ; plus préférentiellement choisie parmi les oxydes métalliques ; plus particulièrement choisie parmi les oxydes de fer, et encore plus particulièrement choisie parmi les oxydes de fer noirs (CI 77499).

24. Composition selon la revendication 23, où la ou les matière(s) colorante(s) est(sont) présente(s) en une teneur allant de 1,0 à 30,0 % en poids, de préférence de 3,0 % à 25,0 % en poids, plus particulièrement de 4,0 à 15,0 % en poids par rapport au poids total de la composition.

25. Composition selon l'une quelconque des revendications précédentes, comprenant en plus au moins un polymère filmogène hydrophobe ou lipophile, de préférence hydrophile, et plus particulièrement un homopolymère de vinylpyrrolidone.

26. Composition selon l'une quelconque des revendications précédentes sous la forme d'un produit pour les cils tel qu'un mascara, d'un produit pour les sourcils ou d'un produit pour le contour des yeux tel qu'un eye liner.

27. Ensemble, ou kit, de conditionnement et d'application d'une composition cosmétique de revêtement des fibres kératiniques comprenant :

   - un dispositif de conditionnement comprenant la composition telle que définie dans l'une quelconque des revendications précédentes ;
   - un applicateur de ladite composition.

28. Procédé de revêtement des matières kératiniques, en particulier la peau, notamment le contour des yeux, des cils ou des sourcils, des fibres kératiniques telles que les cils et/ou les sourcils, comprenant une étape d'application sur lesdites matières kératiniques d'au moins une composition telle que définie selon l'une quelconque des revendications 1 à 26.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1920759 A1
- FR 0853634
- US 2004175338 A
- US 4578266 A
- FR 2679771
- EP 1184426 A
- US 5188899 A

**Non-patent literature cited in the description**

- The HLB System. A time-saving guide to Emulsifier Selection. ICI Americas Inc, 1984
- **GRIFFIN**. *J. Soc. Cosm. Chem.*, 1954, vol. 5, 249-256
- Ullmann's Encyclopedia of Industrial Chemistry. Wiley-VCH Verlag GmbH & Co. KGaA, 2015
- Pigments, Inorganic. Ullmann's Encyclopedia
- **VAN DE HULST, H.C.** Light Scattering by Small Particles. Wiley, 1957